(19) Europäisches Patentamt / European Patent Office / Office européen des brevets

(11) **EP 3 904 068 A2**

(12) **EUROPEAN PATENT APPLICATION**
published in accordance with Art. 153(4) EPC

(43) Date of publication:
**03.11.2021 Bulletin 2021/44**

(21) Application number: **19904083.3**

(22) Date of filing: **27.12.2019**

(51) Int Cl.:
**B32B 5/02** (2006.01)   **H01B 1/20** (2006.01)
**H01B 5/14** (2006.01)   **H01B 13/00** (2006.01)
**H01B 7/06** (2006.01)   **A61B 5/0408** (2006.01)
**A61B 5/0478** (2006.01)   **B32B 7/022** (2019.01)
**B32B 7/025** (2019.01)   **H05K 1/02** (2006.01)
**H05K 1/09** (2006.01)   **H05K 3/12** (2006.01)

(86) International application number:
**PCT/JP2019/051558**

(87) International publication number:
**WO 2020/138477 (02.07.2020 Gazette 2020/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.12.2018   JP 2018245695
27.12.2018   JP 2018245694
27.12.2018   JP 2018245696**

(71) Applicant: **TOYOBO CO., LTD.
Osaka-shi
Osaka 530-8230 (JP)**

(72) Inventors:
• **NAKAMURA, Makoto**
  **Otsu-shi, Shiga 520-0292 (JP)**
• **IRIE, Michihiko**
  **Otsu-shi, Shiga 520-0292 (JP)**
• **KONDO, Takashi**
  **Otsu-shi, Shiga 520-0292 (JP)**
• **NARUSAWA, Haruhiko**
  **Otsu-shi, Shiga 520-0292 (JP)**

(74) Representative: **Müller-Boré & Partner
Patentanwälte PartG mbB
Friedenheimer Brücke 21
80639 München (DE)**

(54) **CONDUCTIVE PASTE FOR FORMING STRETCHABLE CONDUCTOR, STRETCHABLE CONDUCTOR LAYER, METHOD FOR PRODUCING STRETCHABLE CONDUCTOR LAYER, STRETCHABLE ELECTRICAL WIRING STRUCTURE, AND BIOMETRIC INFORMATION MEASURING DEVICE**

(57)   The purpose of the present invention is to provide a conductive paste for forming a stretchable conductor having washing durability. The conductive paste for forming a stretchable conductor of the present invention includes conductive particles and a flexible resin, wherein the flexible resin has a water content after being immersed in water of 40°C for 24 hours of 5% by mass or less, and wherein the flexible resin is contained in an amount of 13% by mass to 35% by mass relative to total solids content in the conductive paste for forming a stretchable conductor.

**EP 3 904 068 A2**

**Description**

TECHNICAL FIELD

The present invention relates to a garment-type biological information measurement device. More particularly, the present invention relates to a garment-type biological information measurement device including an electrode and wiring including a coating film of a stretchable conductor that is obtained from a conductive paste for forming a stretchable conductor including conductive particles and a flexible resin and that has stretching characteristics and excellent washing durability.

BACKGROUND ART

Recently, a wearable electronic device intended to use an electronic device having input/output function, calculation function, and communication function in a state of being very close to or in close contact with a body has been developed. As such a wearable electronic device, devices with an accessory-type shape such as a wristwatch, eyeglasses, and earphones, and a textile-integrated device where electronic functions are incorporated into a garment are known.

With a view to a use for a wiring in the textile-integrated device, a conductive film that does not lose electrical continuity even when stretched has been developed by focusing on the type and combination of conductive particles (Patent Documents 1 and 2).

In particular, in order not only to maintain electrical continuity during stretching but also to enable measurement with a low resistance value even during stretching, the stretching properties and electrical characteristics of the conductive layer have been optimized (Patent Document 2). Further, from the viewpoint of control of a coating film structure such as segregation of conductive particles in a conductive coating film, attempts have been made to suppress deterioration of resistance during elongation (Patent Documents 3 and 4).

RELATED ART DOCUMENTS

PATENT DOCUMENTS

Patent Document 1: JP-B-5363592
Patent Document 2: JP-B-5486268
Patent Document 3: WO 2017/154726
Patent Document 4: WO 2015/119217

DISCLOSURE OF THE INVENTION

PROBLEMS TO BE SOLVED BY THE INVENTION

A conductive paste used to form a stretchable conductor is required to have printability according to a printing method. A printing method mainly used for forming a pattern of the conductive paste is a screen printing or a gravure printing. The use of natural rubber or synthetic rubber as a flexible resin serving as a binder resin for a conductive paste for forming a stretchable conductor has already been attempted widely. The viscosity typical of such rubber materials has a favorable effect on the extensibility of a conductive layer and the adhesiveness of a conductive layer to a substrate, whereas from the viewpoint of printability, the viscosity often adversely affects the transfer efficiency, especially when the paste is transferred from a printing plate to a substrate to be printed. That is, the rubber viscosity may significantly impair plate separation.

From this viewpoint, there is a demand for a conductive paste for forming a stretchable conductor that can be preferably applied to a printing method, such as screen printing and gravure printing, in which paste (ink) is transferred by direct contact.

In the case where a layer (stretchable conductor layer) composed of a stretchable conductor obtained from such a conductive paste is used as an electrical wiring or a skin contact electrode material for a garment-type textile-integrated device, in response to a stretch deformation of the garment during wearing, the stretchable conductor layer is also required to deform by following the stretch deformation.

In addition, it is empirically known that a greater stretch deformation occurs upon putting on and taking off the garment than a stretch deformation during wearing, and the stretchable conductor layer is also required to have sufficient stretchability.

Furthermore, when the stretchable conductor layer is applied to a garment-type textile-integrated device, the stretchable conductor layer is required to have resistances based on the assumption of contact with not only water but also liquids of biological origin such as sweat, saliva, tears, urine, and blood, or seawater.

In the case where the stretchable conductor layer is applied to a garment-type textile-integrated device, washing durability is a very important required characteristic. At the time of washing, the stretchable conductor layer is immersed in water containing a detergent and randomly undergoes deformations such as stretching, compression, bending, and twisting due to stirring or the like during washing and in the process from dehydration to drying.

Water containing a detergent, that is, a surfactant, has a small contact angle with a solid surface and easily penetrates into a very narrow gap. If there are cracks in the stretchable conductor layer, penetration of water containing a detergent into the layer is unavoidable.

Although there is a significant regional difference in a temperature during washing, it should be noted that warm water of about 40°C may be used. Furthermore, it must be borne in mind that a temperature inside a dryer may become about 80°C and exceeds glass transition temperatures of many flexible resins such as rubber materials. Exposure to direct sunlight should be considered even in the case of natural drying.

MEANS FOR SOLVING THE PROBLEMS

As described above, the conductive paste for forming a stretchable conductor layer used to form an electrode and wiring in a biological information measurement garment is required to have printability, and in addition, the formed stretchable conductor layer is required to have various characteristics such as resistance to liquids of biological origin, durability against a detergent, and mechanical durability, heat resistance, and light resistance during the processes from washing to drying.

The inventors made intensive studies in light of the situation above, and as a result, have arrived at the invention having the following configurations.

[1] A conductive paste for forming a stretchable conductor, the conductive paste including at least conductive particles and a flexible resin,

wherein the flexible resin has a saturated water content of 5% by mass or less, and
wherein the flexible resin is contained in an amount of 13% by mass to 35% by mass relative to total solids content in the conductive paste for forming a stretchable conductor.

[2] The conductive paste for forming a stretchable conductor according to above [1], wherein the flexible resin is a non-crosslinked polyurethane resin.

[3] The conductive paste for forming a stretchable conductor according to above [1] or [2], wherein the flexible resin has a glass transition temperature of —10°C or higher and +10°C or lower.

[4] A stretchable conductor layer obtained by printing or applying the conductive paste for forming a stretchable conductor according to any one of above [1] to [3] on or to a substrate, followed by drying,

wherein an elongation at break ($\varepsilon'$) in a water-saturated state at 40°C of the stretchable conductor layer is 500% or more.

[5] The stretchable conductor layer according to above [4],

wherein an elongation at break ($\varepsilon$) in a state of equilibrium moisture absorption of the stretchable conductor layer and the elongation at break ($\varepsilon'$) in a water-saturated state at 40°C of the stretchable conductor layer satisfy a relationship of $\varepsilon' / \varepsilon > 0.5$.

[6] A method for producing a stretchable conductor layer, including:

printing or applying the conductive paste for forming a stretchable conductor according to any one of above [1] to [3] on or to a stretchable substrate, followed by drying at a temperature of at least 70°C or higher and 120°C or lower, to obtain a stretchable conductor layer having a saturated water content of 5% by mass or less.

[7] A method for producing a stretchable conductor layer, including:

printing or applying the conductive paste for forming a stretchable conductor according to any one of above [1] to [3] on or to a non-stretchable release substrate, followed by drying at a temperature of at least 70°C or higher and 120°C or lower, to obtain a stretchable conductor layer having a saturated water content of 5% by mass or less.

[8] A stretchable electrical wiring structure including: at least a substrate including a fiber structure, a stretchable electrode configured to directly contact a living body surface, a stretchable electrical wiring, and a stretchable insulating layer,

wherein the stretchable insulating layer includes a flexible resin composition having a saturated water content of 5% by mass or less and an elastic modulus of 10 MPa or more and 1000 MPa or less when saturated with water.

[9] The stretchable electrical wiring structure according to above [8], wherein the stretchable insulating layer is formed on a side facing a living body surface of the stretchable electrical wiring structure.

[10] The stretchable electrical wiring structure according to above [8] or [9], wherein the stretchable insulating layer is formed between the stretchable electrode and/or the stretchable electrical wiring and the substrate including the fiber structure.

[11] The stretchable electrical wiring structure according to any one of above [8] to [10], wherein the stretchable insulating layer is formed on a surface opposite to a surface, of the substrate including the fiber structure, on which the stretchable electrode and/or the stretchable electrical wiring are formed.

[12] The stretchable electrical wiring structure according to any one of above [8] to [11], wherein a stretchable conductor layer constituting the stretchable electrode and/or the stretchable electrical wiring has a saturated water content of 5% by mass or less.

[13] The stretchable electrical wiring structure according to any one of above [8] to [12],

wherein the stretchable conductor layer includes conductive particles and a flexible resin,
wherein the flexible resin has a saturated water content of 5% by mass or less, and
wherein the flexible resin is contained in an amount of 13% by mass to 35% by mass relative to the stretchable conductor layer.

[14] The stretchable electrical wiring structure according to any one of above [8] to [13], wherein the flexible resin has a glass transition temperature of —10°C or higher and 10°C or lower.

[15] The stretchable electrical wiring structure according to any one of above [8] to [14], wherein an elongation at break ($\varepsilon'$) in a water-saturated state at 40°C of the stretchable insulating layer is 500% or more.

[16] The stretchable electrical wiring structure according to any one of above [8] to [15], wherein an elongation at break ($\varepsilon$) in a state of equilibrium moisture absorption of the stretchable insulating layer and the elongation at break ($\varepsilon'$) in a water-saturated state at 40°C of the stretchable insulating layer satisfy a relationship of $\varepsilon' / \varepsilon > 0.5$.

[17] The stretchable electrical wiring structure according to any one of above [8] to [16], wherein the flexible resin is a non-crosslinked polyurethane resin.

[18] The stretchable electrical wiring structure according to any one of above [8] to [17], wherein the flexible resin is a polyester-based or polycarbonate-based polyurethane resin containing 13% by mass or more of an aromatic group in a resin skeleton.

[19] A biological information measurement device including a stretchable electrode configured to directly contact a living body surface,
wherein a stretchable conductor layer constituting the stretchable electrode is the stretchable conductor layer according to above [4] or [5].

[20] The biological information measurement device according to above [19], wherein a stretchable electrical wiring is formed by a same stretchable conductor layer as the stretchable conductor layer constituting the stretchable electrode.

[21] The biological information measurement device according to above [19],
wherein the biological information measurement device includes a conductive protective film including a carbon-based filler as a conductive component on a surface of the stretchable electrode.

[22] The biological information measurement device according to any one of above [19] to [21],
wherein the biological information measurement device includes a layer including a flexible resin on a surface opposite to a contact surface with a living body surface of the stretchable electrode and/or the stretchable electrical wiring, the flexible resin having a saturated water content of 5% by mass or less and an elastic modulus of 10 MPa or more and 1000 MPa or less in a range of water content of 0 to 5% by mass.

[23] The biological information measurement device according to any one of above [20] to [22],
wherein the biological information measurement device includes an insulating layer including a flexible resin on a contact surface with a living body surface of the stretchable electrical wiring, the flexible resin having a saturated water content of 5% by mass or less and an elastic modulus of 10 MPa or more and 1000 MPa or less in a range of water content of 0 to 5% by mass.

Preferably, the present invention further includes the following configurations.

[24] A method for producing a biological information measurement device having a stretchable conductor layer, including at least the following steps of:

(1) forming a stretchable conductor layer by printing a paste for forming a stretchable conductor on a release sheet, followed by drying;
(2) forming a hot-melt adhesive layer on a surface opposite to the release sheet of the stretchable conductor layer; and

(3) transferring the stretchable conductor layer to a textile via the hot-melt layer.

[25] The conductive paste for forming a stretchable conductor according to any one of the above [1] to [3], the stretchable electrical wiring structure according to the above [8] to [18], and the biological information measurement device according to the above [19] to [23], wherein the flexible resin does not contain an ether bond, a hydroxyl group, a carboxylic acid group, a sulfonic acid group, and an amino group in the resin skeleton.

[26] The conductive paste for forming a stretchable conductor according to any one of the above [1] to [3], the stretchable electrical wiring structure according to the above [8] to [18], and the biological information measurement device according to the above [19] to [23], wherein the flexible resin contains at least one component selected from a siloxane bond, a fluoroalkyl group, an alkyl group, and a phenyl group in the resin skeleton.

[27] The conductive paste for forming a stretchable conductor according to any one of the above [1] to [3], the stretchable electrical wiring structure according to the above [8] to [18], and the biological information measurement device according to the above [19] to [23], wherein the flexible resin is a polyester-based or polycarbonate-based polyurethane resin containing 13% by mass or more of an aromatic group in the resin skeleton.

[28] The conductive paste for forming a stretchable conductor according to any one of the above [1] to [3], the stretchable electrical wiring structure according to the above [8] to [18], and the biological information measurement device according to the above [19] to [23], wherein the flexible resin is a polyester-based or polycarbonate-based polyurethane resin containing 40% by mass or more of an aromatic group in the resin skeleton.

EFFECTS OF THE INVENTION

The effect of the present invention is that the conductive paste for forming an electrode and/or wiring of the biological information measurement device of the present invention has excellent printability, and a stretchable conductor layer formed from the conductive paste has improved various resistance properties, particularly improved washing durability.

According to the present invention, the stretchable conductor layer can maintain sufficient washing durability even in a thin film region having a thickness of 50 $\mu$m or less, preferably 25 $\mu$m or less, and more preferably 15 $\mu$m or less, and further can maintain washing durability even in a fine line region having a printed line width of 3 mm or less, preferably 1.2 mm or less, and more preferably 0.8 mm or less.

The inventors have found that performance degradation during a washing process depends on the water content of the coating film, particularly the water content of the flexible resin serving as a binder resin. When a resin material absorbs water, it exhibits physical properties different from those in dry state. Therefore, to achieve durability against mechanical movements such as stretching and friction under a washing environment and other environments of immersion in water for a long time, it is necessary to consider control of physical properties based on the state of the coating film under a washing environment. The above-mentioned patent documents do not describe the physical properties of the coating film containing water, and the control of the physical properties from such a standpoint has not been performed in past developments.

Moisture generally acts as a plasticizer for a resin, and thus may increase the flexibility of the resin under conditions in near static state, resulting in a positive effect on simple bending and the like. However, during the washing process, both a stretching load and a compression load are complexly applied to the stretchable conductor layer, and it is difficult to understand by comparison with a simple system such as simple bending.

In particular, a phenomenon is observed in which moisture absorbed on the compression load side is squeezed out of a resin portion. The squeezed moisture easily exudes to different material interfaces in the materials, and in the case of a mixture of organic and inorganic materials such as a cured product of a conductive paste, it is presumed that moisture exudes to the interface between the conductive particles and the binder resin, causing peeling of the both, and at the same time, causing inhibition of electrical contact between the conductive particles.

Especially when an application to a garment-type textile-integrated device that uses a wiring that is small in thickness or small in line width is considered, in a wiring that is small in film thickness or line width of a conductive layer, the contact between conductive particles is easily broken completely even by relatively minute mechanical damages, causing a large influence on the entire conductive circuit.

In the present invention, by suppressing the saturated water content of the flexible resin serving as a binder resin to 5% by mass or less, the above-described exudation of moisture to the interface during compression is suppressed, whereby the stretchable conductor layer has been realized which is excellent in mechanical strength and elongation, has low water absorption, and therefore maintains coating film strength and stretchability above a certain level even under an environment assuming washing. As a result, it has become possible to manifest excellent washing durability even in the stretchable conductor layer with a small film thickness and small line width. The stretchable conductor layer of the present invention can stably maintain electrical conductivity as an electrical wiring even after being repeatedly washed 100 times or more.

Further, in the present invention, it has been found that the water content of the stretchable insulating layer used in combination with the stretchable conductor layer also strongly affects the washing durability. By suppressing the saturated water content of the flexible resin that forms the stretchable insulating layer to 5% by mass or less, the above-described exudation of moisture to the interface during compression is suppressed, whereby the stretchable electrical wiring structure has been realized which is excellent in mechanical strength and elongation, has low water absorption, and therefore maintains durability above a certain level even under an environment assuming washing when combined with the stretchable conductor layer. As a result, it has become possible to manifest excellent washing durability even in the stretchable conductor layer with a small film thickness and small line width. The stretchable electrical wiring structure in which the stretchable conductor layer and the stretchable insulating layer of the present invention are combined can stably maintain electrical conductivity as an electrical wiring even after being repeatedly washed 100 times or more.

BRIEF DESCRIPTION OF THE DRAWINGS

FIG. 1 is a schematic cross-sectional view showing a case in which no electrode surface layer is provided in an electrode and electrical wiring obtained by a printing method.
FIG. 2 is a schematic cross-sectional view showing a case in which an electrode surface layer is provided in an electrode and electrical wiring obtained by a printing method.
FIG. 3 is a schematic cross-sectional view showing a case in which no electrode surface layer is provided in an electrode and electrical wiring obtained by a printing transfer method.
FIG. 4 is a schematic cross-sectional view showing a case in which an electrode surface layer is provided in an electrode and electrical wiring obtained by a printing transfer method.

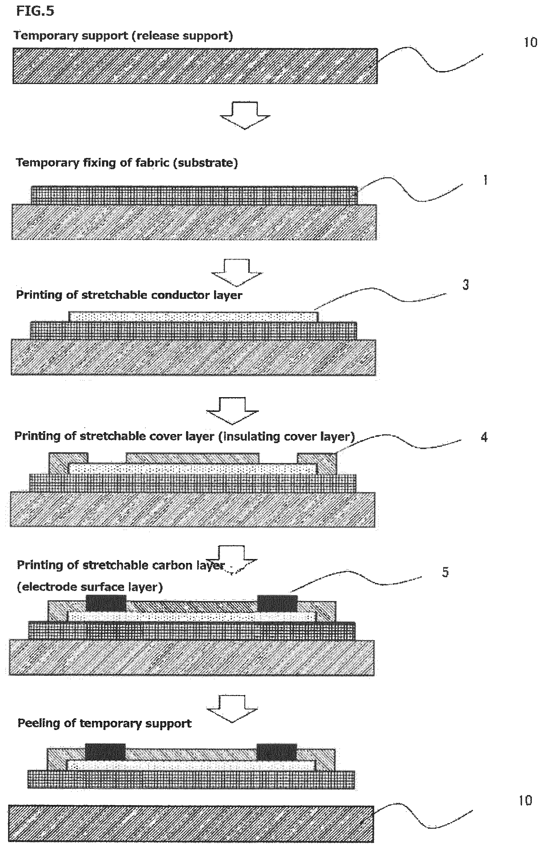

FIG. 5 is a schematic process view showing a production method of an electrode and electrical wiring by a printing method.
FIG. 6 is a schematic process view showing an example of a production method of an electrode and electrical wiring by a printing transfer method.
FIG. 7 is a schematic view showing an example of each planar shape of an insulating base layer, a stretchable conductor layer, a stretchable cover layer, and an electrode protective layer that constitute a stretchable electrode and a stretchable wiring portion for measuring an electrocardiogram.
FIG. 8 is a schematic view showing an image of a T-shirt in which a stretchable electrode and a stretchable wiring portion for measuring an electrocardiogram are arranged, that is, a biological information measurement device. The stretchable electrode and the stretchable wiring portion are arranged on the inside of the T-shirt in actuality.

MODE FOR CARRYING OUT THE INVENTION

The present invention is directed to a conductive paste for forming a stretchable conductor, a stretchable conductor layer, a method for producing a stretchable conductor layer, a stretchable electrical wiring structure, and a biological information measurement device. The conductive paste for forming a stretchable conductor is applied to a substrate, and dried and cured to form a stretchable conductor layer. The stretchable conductor layer is combined with a stretchable insulating layer to form a stretchable electrical wiring structure. The biological information measurement device uses the stretchable electrical wiring structure as an electrical wiring and a living-body contact electrode.

The conductive paste for forming a stretchable conductor of the present invention includes at least conductive fine particles, a flexible resin, and a solvent. The conductive particles included in the conductive paste for forming a stretchable conductor are fine particles of a conductive material selected from metals, carbon-based materials, conductive polymers, and the like, and preferably metal powder containing at least silver or a silver alloy.

Preferred shapes of the metal powder include known scales (also known flakes), spheres, dendrites, aggregates (a shape wherein spherical primary particles are aggregated into a three-dimensional shape), and the like.

In the conductive paste for forming a stretchable conductor of the present invention, it is preferable to mainly use flaky silver particles or amorphous aggregated silver powder. Here, "mainly use" means to use it at 90% by mass or more relative to the conductive particles. The amorphous aggregated powder is a three-dimensional aggregate of spherical or irregular primary particles. The amorphous aggregated powder and flaky powder are preferable because they have a specific surface area larger than that of spherical powder and the like and can form a conductive network even with a low filling amount. The flaky powder is further preferable because a coating film having excellent stretchability can be obtained by using the flaky powder, so that damage to the coating film caused by stretching does not easily progress.

Although there is no particular limitation for the particle diameter of the flaky powder, the average particle diameter (50% D) measured by a dynamic light scattering method is preferably 0.5 to 20 $\mu$m, and more preferably 3 to 12 $\mu$m. If the average particle diameter exceeds 15 $\mu$m, the formation of a fine wiring may become difficult, and clogging occurs in the case of screen printing or the like. If the average particle diameter is less than 0.5 $\mu$m, the particles cannot contact with each other when the filling amount is small, and as a result, the electrical conductivity may deteriorate.

The conductive paste for forming a stretchable conductor of the present invention may contain other conductive powders or non-conductive particles in addition to the silver powder described above as long as the contents of the invention are not impaired. Examples of the conductive powders include silver powder with a shape other than amorphous aggregated powder and flaky powder; noble metal powder such as gold powder, platinum powder, and palladium powder; base metal powder such as copper powder, nickel powder, aluminum powder, and brass powder; plated powder wherein heterologous particles including base metal, inorganic substances such as silica, and the like are plated with noble metal such as silver; base metal powder that is made into alloy using noble metal such as silver; graphite; and carbon powder such as carbon black. Those conductive powders may be used singly or in combination of two or more thereof.

In the present invention, barium sulfate particles can be used as the non-conductive particles. As the barium sulfate particles used in the present invention, ground barite obtainable by grinding a barite mineral called a natural barite, and a so-called precipitated barium sulfate produced by a chemical reaction can be used. It is preferred in the present invention to use the precipitated barium sulfate of which particle diameter is easily controlled. The average particle diameter of the barium sulfate particles preferably used, as determined by a dynamic light scattering method, is preferably 0.01 to 18 $\mu$m, more preferably 0.05 to 8 $\mu$m, and further preferably 0.2 to 3 $\mu$m. In addition, the barium sulfate particles in the present invention are preferably subjected to a surface treatment with hydroxide and/or oxide of one or both of Al and Si. By such a surface treatment, the hydroxide and/or oxide of one or both of Al and Si adhere to the surface of the barium sulfate particles. The adhering amount of these compounds is preferably 0.5 to 50, and more preferably 2 to 30 relative to 100 of barium elements at an element ratio detected by X-ray fluorescence analysis.

The conductive paste for forming a stretchable conductor includes the above-described silver particles and a flexible resin. The flexible resin can be contained in an amount of 13 to 35% by mass relative to the mass of the total solids content in the silver particles and the flexible resin. In the present invention, the flexible resin is preferably contained in an amount of 14 to 30% by mass or less, more preferably 15 to 27% by mass, and further preferably 15 to 22% by mass or less, relative to the mass of the total solids content in the silver particles and the flexible resin. By allowing the amount of the flexible resin to be within the predetermined range, it is possible to achieve both high washing durability and necessary and sufficient electrical conductivity.

The flexible resin used as a raw material for the conductive paste for forming a stretchable conductor in the present invention is a resin having an elastic modulus of 1 to 1000 MPa. Examples of such a flexible resin include a thermoplastic resin, a thermosetting resin, and rubber. In order to develop the film stretchability, natural rubber, synthetic rubber, urethane resin and the like can be preferably used. Examples of the synthetic rubber include urethane rubber, acrylic rubber, silicone rubber, butadiene rubber, rubber containing a nitrile group such as nitrile rubber or hydrogenated nitrile rubber, isoprene rubber, vulcanized rubber, styrene-butadiene rubber, butyl rubber, chlorosulfonated polyethylene rubber, ethylene propylene rubber, vinylidene fluoride copolymer, and the like. The range of the elastic modulus in the present invention is preferably 1 to 600 MPa, more preferably 1 to 500 MPa, and still more preferably 3 to 300 MPa. Among these, urethane resin can be most preferably used.

The urethane resin used in the present invention can be obtained by reacting a soft segment composed of a polyether-based, polyester-based, or polycarbonate-based polyol with a hard segment composed of diisocyanate or the like. As the soft segment component, polyester polyol is more preferable because of the degree of freedom in molecular design.

Examples of the polyether polyol used in the present invention include polyalkylene glycols such as polyethylene glycol, polypropylene glycol, polypropylene triol, polypropylene tetraol, polytetramethylene glycol, polytetramethylene triol, a copolymer obtained by copolymerizing a monomer material such as cyclic ether for synthesizing these, derivatives and modified products obtained by introducing a side chain or a branched structure to these, and mixtures thereof. Among these, polytetramethylene glycol is preferred. The reason is that the mechanical properties are excellent.

As the polyester polyol used in the present invention, there can be used aromatic-based polyester polyol, aromatic/aliphatic copolymer polyester polyol, aliphatic polyester polyol, and alicyclic polyester polyol. As the polyester polyol in the present invention, either a saturated type or an unsaturated type may be used. Among these, aliphatic polyester polyol is preferred.

Commercially available products can also be used as the aliphatic polyester polyol. Specific examples of the commercially available products include, for example, Polylite ODX-688, ODX-2044, and ODX-240 (manufactured by DIC Corporation), Kuraray Polyol P-2010, P-2050, and P-1010 (Kuraray), Teslack 2461, 2455, and 2469 (manufactured by Hitachi Chemical Company), and the like.

Examples of the polycaprolactone diol used in the present invention include polycaprolactone diol compounds obtained by ring-opening addition reaction of lactones such as γ-butyllactone, ε-caprolactone, and δ-valerolactone.

Examples of commercially available products of polycarbonate diol compounds used in the present invention include Kuraray Polyol C series manufactured by Kuraray Co., Ltd., Duranol series manufactured by Asahi Kasei Chemicals Corporation, and the like. Specific examples thereof include Kuraray Polyol C-1015N, Kuraray Polyol C-1065N, Kuraray Polyol C-2015N, Kuraray Polyol C2065N, Kuraray Polyol C-1050, Kuraray Polyol C-1090, Kuraray Polyol C-2050, Kuraray Polyol C-2090, DURANOL-T5650E, DURANOL-T5651, and DURANOL-T5652.

Examples of the diisocyanate compound used in the present invention include aromatic diisocyanates such as 2,4-tolylene diisocyanate, 2,6-tolylene diisocyanate, p-phenylene diisocyanate, 4,4'-diphenylmethane diisocyanate, m-phenylene diisocyanate, 3,3'-dimethoxy-4,4'-biphenylene diisocyanate, 2,6-naphthalene diisocyanate, 3,3'-dimethyl-4,4'-biphenylene diisocyanate, 4,4'-diphenylene diisocyanate, 4,4'-diisocyanate diphenyl ether, 1,5-naphthalene diisocyanate, and m-xylene diisocyanate; aliphatics of 1,6-hexane diisocyanate, isophorone diisocyanate, 4,4'-diphenylmethane diisocyanate, and hydrogenated xylylene diisocyanate (ortho, meta, para); and alicyclic diisocyanate. Among these, 4,4'-diphenylmethane diisocyanate, 2,4-tolylene diisocyanate, 2,6-tolylene diisocyanate, and isophorone diisocyanate are preferable. Moreover, as necessary, these isocyanates may be used in combination of two or more thereof, or may be used in combination with a tri- or more functional polyisocyanate.

As necessary, the polyurethane resin used in the present invention may be copolymerized with a diol compound or the like generally called a chain extender.

Examples of the diol compound used as a chain extender include aliphatic glycols such as ethylene glycol, diethylene glycol, triethylene glycol, propylene glycol, dipropylene glycol, tripropylene glycol, 1,3-propanediol, 2-methyl-1,3-propanediol, 2-methyl-2-propyl-1,3-propanediol, 2-butyl-2-ethyl-1,3-propanediol, 2-butyl-2-hexyl-1,3-propanediol, 1,2-butanediol, 1,3-butanediol, 1,4-butanediol, 2,3-butanediol, 1,5-pentanediol, 2-methyl-2,4-pentanediol, 3-methyl-1,5-pentanediol, 2,2,4-trimethyl-1,3-pentanediol, neopentyl glycol, 1,6-hexanediol, 2-ethyl-1,3-hexanediol, 2,5-dimethyl-2,5-hexanediol, 1,8-octanediol, 2-methyl-1,8-octanediol, and 1,9-nonanediol. Alternatively, low molecular weight triols such as trimethylolpropane and triethanolamine, diamine compounds such as diethylamine and 4,4'-diaminodiphenylmethane, and trimethylolpropane can be given. Among these, 1,6-hexanediol is particularly preferable.

When a polyurethane resin is produced, stannous octylate, dibutyltin dilaurate, triethylamine, bismuth metal, or the like may be used as a catalyst.

The glass transition temperature of the flexible resin used in the present invention is preferably 10°C or lower, more preferably -20°C or higher and 10°C or lower, most preferably -10°C or higher and 10°C or lower. When the flexible resin has a glass transition temperature within the above range, the produced conductive coating film has a high elongation, so that a frequency of occurrence of cracking during washing decreases, and a change in resistance when elongated reduces.

The flexible resin used in the present invention preferably has a saturated water content of 5% or less. The saturated water content represents a water content in which a change of the water content shows less than 10% of the value on the previous day when the water content is examined for each next day. This is because a flexible resin having lower water absorption is less likely to be plasticized and hydrolyzed by water. This allows the occurrence and progress of cracking in the stretchable conductor layer to be suppressed in a mode in which stretching movement is performed in water such as washing. In addition, poor conductivity caused by swelling of the flexible resin due to water contained therein can be avoided.

The saturated water content of 5% of the flexible resin in the present invention can be achieved by combining known techniques. For example, the reduction of a hydrophilic component such as an ether bond, a hydroxyl group, a carboxylic acid group, a sulfonic acid group, and an amino group in the resin skeleton, and the introduction of a hydrophobic component such as a siloxane bond, a fluoroalkyl group, and an aromatic component such as an alkyl group and a phenyl group into the resin skeleton are effective techniques. The densification and the increase in crystallinity of the resin are also effective techniques. Among these, in consideration of price and flexibility, the introduction of an aromatic component into a polyester-based or polycarbonate-based polyurethane resin skeleton is preferable.

Specifically, by allowing the aromatic concentration in the polyester-based or polycarbonate-based polyurethane resin skeleton to be 10% by mass or more, more preferably 20% by mass or more, and most preferably 30% by mass or more, the saturated water content can be 5% or less while maintaining flexibility.

It is desirable to add a rheology control agent to the conductive paste for forming a stretchable conductor of the present invention to improve the printability of the paste. A combination of an acrylic rheology control agent and a urethane resin is further desirable because such a combination leads to improvement in flexibility due to plasticization of the coating film and formation of a segregation structure, resulting in improved washing durability.

In addition to the rheology control agent, it is also possible to blend a thixotropic property imparting agent, antifoaming agent, flame retardant, tackifier, preventing agent for hydrolysis, plasticizer, antioxidant, ultraviolet absorber, flame retardant, pigment, or dye.

The conductive paste for forming a stretchable conductor of the present invention may contain an organic solvent in addition to the above-mentioned conductive particles and flexible resin. The organic solvent used in the present invention preferably has a boiling point of 100°C or higher and lower than 300°C, and more preferably 130°C or higher and lower than 280°C. When the boiling point of the organic solvent is too low, the solvent may volatilize during the paste production process and use of the paste, and there is concern that the component ratio of the conductive paste will be apt to change. On the other hand, when the boiling point of the organic solvent is too high, the amount of solvent remaining in the dried and cured coating film becomes large, and hence there is concern that the reliability of the coating film will deteriorate.

Specific examples of the organic solvent using in the present invention include cyclohexanone, toluene, xylene, isophorone, y-butyrolactone, benzyl alcohol, Solvesso 100, 150 and 200 (manufactured by Exxon Chemical), propylene glycol monomethyl ether acetate, terpineol, butyl glycol acetate, diamylbenzene, triamylbenzene, n-dodecanol, diethylene glycol, ethylene glycol monoethyl ether acetate, diethylene glycol monoethyl ether acetate, diethylene glycol monobutyl ether acetate, diethylene glycol dibutyl ether, diethylene glycol monoacetate, triethylene glycol diacetate, triethylene glycol, triethylene glycol monomethyl ether, triethylene glycol monoethyl ether, triethylene glycol monobutyl ether, tetraethylene glycol, tetraethylene glycol monobutyl ether, tripropylene glycol, tripropylene glycol monomethyl ether, and 2,2,4-trimethyl-1,3-pentanediol monoisobutyrate. As to a petroleum hydrocarbon, there may be exemplified AF Solvent No. 4 (boiling point: 240 to 265°C), No. 5 (boiling point: 275 to 306°C), No. 6 (boiling point: 296 to 317°C), and No. 7 (boiling point: 259 to 282°C), and No. 0 Solvent H (boiling point: 245 to 265°C) manufactured by Nippon Oil Corporation. Note that the organic solvent may be used singly, or in combination of two or more thereof. Such organic solvents are appropriately contained such that a conductive paste has a viscosity suitable for coating, printing, or the like.

The conductive paste for forming a stretchable conductor for forming the stretchable conductor layer of the present invention can be prepared by mixing and dispersing conductive particles, barium sulfate particles, a stretchable resin, and a solvent as materials using a dispersing machine such as a dissolver, a three-roll mill, a self-revolving mixer, an attritor, a ball mill, or a sand mill.

The stretchable conductor layer of the present invention can be obtained by applying or printing the conductive paste for forming a stretchable conductor to or on a substrate, followed by drying. It is preferred that the conductive paste for forming a stretchable conductor of the present invention be capable of forming a stretchable conductor layer having a specific resistance of less than $1.0 \times 10^{-3}$ ($\Omega \cdot$cm). When the specific resistance is $1.0 \times 10^{-3}$ ($\Omega \cdot$cm) or more, in a designing of a stretchable wiring, a stretchable antenna and a stretchable electrode which are used in the fields of an FPC, a robot, a smart wear, a health care sensor, a display, a solar battery, an RFID and a game machine, restrictions in a film thickness, a wiring length, a wiring width, and the like may be resulted, and an adaptation is not possible. In addition, when a rise in the specific resistance upon elongation and after a repetitive stretching is taken into consideration, the specific resistance of the coating film is more preferably less than $5.0 \times 10^{-4}$ ($\Omega \cdot$cm).

Further, the stretchable conductor layer of the present invention preferably has an elongation at break of more than 35%. When adaptation to joints of a human body and a robot is taken into consideration, the elongation at break of the stretchable conductor layer is more preferably 60% or more and, in view of reliability, still more preferably 100% or more.

It is desirable that the stretchable conductor layer have a saturated water content of 5% by mass or less. As to the saturated water content, a water content is measured every 24 hours and determined to reach the saturated water content at the point where the water content has not increased by 1% by mass or more.

A stretchable conductor layer having a lower saturated water content is less likely to have a change in mechanical properties due to plasticization by washing and decomposition of a flexible resin, and can exhibit excellent durability.

Even when the stretchable conductor layer formed from the paste of the present invention is a fine-line thin-film having a thickness of 50 um or less and a line width of 5 mm or less, the stretchable conductor layer can maintain electrical conductivity also after being washed 100 times or more.

Such a stretchable conductor layer has a saturated water content of 5% or less and exhibits an elongation at break of 500% or more when saturated with water. This is because, due to the high strength of resin, the stretchability of the stretchable conductor layer is increased by plasticization while maintaining a coating film strength enough not to break even after containing water, and thus a high elongation at break of 500% or more is exhibited.

If a stretchable conductor layer has an elongation at break of less than 500%, the stretchable conductor layer containing water is presumed to be inferior not only in stretchability but also in strength, and damage such as cracking may occur in the stretchable conductor layer due to stretching movement, water from a sheet surface, and contact with other laundry during washing. Therefore, in a designing of a stretchable wiring and a stretchable electrode which are used in the field of a smart wear, this stretchable conductor layer cannot withstand 100 times-equivalent washing.

The stretchable conductor layer of the present invention, although the range of a film thickness is not particularly limited, preferably has a film thickness of 1 $\mu$m to 1 mm. When the film thickness is less than 1 $\mu$m, defect of the film such as a pinhole is apt to happen and that may result in a problem. When the film thickness is more than 1 mm, the solvent is apt to remain in an inner area of the film and the reproducibility of physical properties of the film may be inferior.

Even when the stretchable conductor layer formed from the conductive paste for forming a stretchable conductor of the present invention is a fine-line thin-film having a thickness of 50 um or less and a line width of 5 mm or less, the stretchable conductor layer can maintain electrical conductivity also after being washed 100 times or more. Such a stretchable conductor layer has a saturated water content of 5% or less, and the ratio of an elongation at break ($\varepsilon$) at

25°C of the stretchable conductor layer in a state of equilibrium moisture absorption and an elongation at break ($\varepsilon$') at 40°C of the stretchable conductor layer when saturated with water is:

$$\varepsilon' / \varepsilon > 0.5.$$

Here, the ratio of specified elongations at break indicates the degree of change in stretchability of the stretchable conductor layer between before and after washing. If this change is 0.5 or less, deterioration in stretchability between before and after washing is large, and it can be considered that the stretchable conductor layer is likely to undergo plasticization by water, or originally has small strength. This stretchable conductor layer may be subject to damage such as cracking during washing due to stretching movement, water from a sheet surface, and contact with other laundry during washing. Therefore, in a designing of a stretchable wiring and a stretchable electrode which are used in the field of a smart wear, the stretchable conductor layer designed as a fine line of 5 mm or less cannot withstand 100 times-equivalent washing.

The electrical wiring of the present invention has, as a first form, a form in which the stretchable conductor paste is printed in a wiring shape on an underlying substrate, and, as a second form, a form in which the stretchable conductor layer is cut out in a wiring shape. The electrical wiring includes an electrode portion of an arbitrary shape and a wiring portion of an arbitrary shape. It is desirable that the electrode portion and the wiring portion be integrally provided as one piece by printing a paste or cutting out a sheet. It is more desirable that a hot-melt agent be provided on the opposite side of the underlying substrate because pressure bonding to a garment is possible.

The underlying substrate to which the conductive paste for forming a stretchable conductor is applied is preferably a flexible or stretchable substrate. Examples of the flexible substrate include paper, cloth, polyethylene terephthalate, polyvinyl chloride, polyethylene, and polyimide. Examples of the stretchable substrate include polyurethane, polydimethylsiloxane (PDMS), nitrile rubber, butadiene rubber, SBS elastomer, and SEBS elastomer. It is preferred that the substrate can be put a crease therein and is stretchable in a planar direction. With this regard, a substrate consisting of rubber or elastomer is preferred.

Although a step for applying the conductive paste for forming a stretchable conductor to the substrate is not particularly limited, it may be conducted, for example, by means of a coating method or a printing method. Examples of the printing method include screen printing method, lithographic offset printing method, ink jet method, flexographic printing method, gravure printing method, gravure offset printing method, stamping method, dispense method, and squeegee printing.

To realize an electrical wiring capable of withstanding 100 times-equivalent washing with a fine line having a width of less than 5 mm designed without a protective layer, the thickness of the electrical wiring is more preferably 30 $\mu$m to 1 mm. By allowing the wiring width to be within this range, high washing durability can be achieved.

When a wiring having a thickness of 30 $\mu$m or less is used, it is more preferable to use a protective layer in combination with the electrical wiring made from the conductive paste for forming a stretchable conductor. The type of the protective layer differs depending on necessary properties such as conductivity, and, for example, an insulating protective layer or a conductive protective layer may be used as appropriate. As examples of covering of the protective layer, not only covering by printing and drying of a paste, but also covering by thermocompression bonding of a film of a flexible resin provided with a hot-melt adhesive can be mentioned.

The electrical wiring of the present invention may be used with a protective layer in order to improve the design quality or impart insulation properties to the wiring portion. By covering with a protective layer having insulation properties, damage from a surface of the wiring can be prevented, and the lifetime as the wiring can be extended. In addition, since the strength of the electrical wiring is enhanced, the durability of the wiring when repeatedly stretched can be improved.

The protective layer can also be used for the purpose of enhancing washing durability. By providing the protective layer, damage to the wiring due to water flow or contact with other laundry during washing can be prevented. This allows even the electrical wiring including a portion having a line width of less than 5 mm and a thickness of less than 30 $\mu$m to maintain electrical conductivity also after 100 times-equivalent washing. More specifically, it is desirable that the electrical wiring be designed such that the line width of the electrode portion is 5 mm or more, and the edge of the protective layer is provided at the portion of the line width of 5 mm or more. Since mechanical load segregation occurs in the vicinity of the boundary of a portion coated with the protective layer, it is desirable to avoid providing the edge of the protective layer in a place having weaker durability in the entire electrical wiring.

The electrical wiring of the present invention may be used with a conductive protective layer using as a raw material carbon or the like in order to improve the design quality or enhance the strength of the electrical wiring. As an example in this case, an example where the entire electrical wiring is covered with a carbon layer to prevent oxidation of silver can be mentioned.

The electrical wiring of the present invention can also be used with a combination of two different types of protective layers, such as a combination of a conductive protective layer and a non-conductive protective layer. Examples of this case include a configuration in which the electrode portion is covered with a conductive protective layer using as a raw material carbon or the like, and the wiring portion is covered with an insulating protective layer such as an insulating coating agent. Two types of protective layers may be partially applied together depending on the design and the degree of difficulty in configuration. Such a configuration is desired in a case where direct contact with the silver electrode is not preferred. The non-conductive protective layer in the present invention is a stretchable insulating layer.

In the present invention, the flexible resins in the respective compositions constituting the stretchable conductor layer, the stretchable insulating layer, and the protective layer may be the same or different. In the present invention, it is preferable that these layers have a common component from the viewpoint of adhesiveness between these layers. The glass transition temperature of the flexible resin is preferably -10°C or higher and +10°C or lower.

To realize an electrical wiring capable of withstanding 100 times-equivalent washing with a fine line having a width of less than 5 mm, it is desirable that the substrate to which the conductive paste for forming a stretchable conductor is applied have a saturated water content of 5% or less. Further, it is preferable that the flexible resin constituting the stretchable insulating layer that is combined with the stretchable conductor layer also have a saturated water content of 5% or less. This is because as the saturated water content is lower, the change in physical properties of the substrate mainly due to plasticization and decomposition by water becomes hard to occur when conducting repetitive washing, so that the adhesion with the wiring can be maintained even after repeated washing.

The flexible resin included in the stretchable insulating layer in the present invention desirably has a saturated water content of 5% or less and an elastic modulus of 10 MPa or more and 1000 MPa or less when saturated with water. Since the physical properties of the substrate are less likely to change between before and after repetitive washing, and a predetermined elastic modulus can be maintained, it is possible to minimize the occurrence and progress of damage such as cracking in the wiring even after long-term washing. As a result, even when the electrical wiring is a fine line, the electrical wiring that maintains excellent conductivity even after 100 times-equivalent washing can be realized.

The elongation at break ($\varepsilon$') at 40°C of the stretchable insulating layer of the present invention in a water-saturated state is preferably 500% or more. It is also preferable that the elongation at break ($\varepsilon$) in a state of equilibrium moisture absorption and the elongation at break ($\varepsilon$') at 40°C in a water-saturated state of the stretchable insulating layer satisfy a relationship of:

$$\varepsilon' / \varepsilon > 0.5.$$

In the present invention, a substrate including a fiber structure, the stretchable electrode and the stretchable electrical wiring that are formed by the stretchable conductor layer and directly contact a living body surface, and further the stretchable insulating layer are combined to obtain a stretchable electrical wiring structure. The stretchable insulating layer is formed on the side facing a living body surface of the stretchable electrical wiring structure. The stretchable insulating layer may be formed between the stretchable electrode and/or the stretchable electrical wiring and the substrate including a fiber structure. Further, the stretchable insulating layer may be formed on the surface opposite to the surface, of the substrate including the fiber structure, on which the stretchable electrode and/or the stretchable electrical wiring are formed.

Further, in the present invention, it is desirable to provide a hot-melt adhesive layer on an adhering surface to the fabric of the stretchable electrical wiring structure. The hot-melt adhesive layer in the present invention is an adhesive composed of a thermoplastic resin capable of adhering to a fabric by heating at a temperature in the range of 45°C to 250°C or a thermosetting resin in an uncured or half-cured state, which is called a reactive hot melt adhesive. The hot-melt adhesive layer in the present invention preferably adheres to a fabric by heating at a temperature preferably in the range of 60°C to 230°C, more preferably in the range of 80°C to 210°C, and further preferably in the range of 90°C to 180°C. By conducting hot-pressing after removal of an unnecessary portion by partial slitting, easy thermocompression bonding is possible. When the stretchable conductor layer having the hot-melt adhesive layer on the opposite side of the substrate is used, the electrical wiring can be transferred to the fabric, and a garment with the electrical wiring can be easily obtained.

The hot-melt adhesive layer can also be provided so as to face the stretchable conductor layer. In this case, the electrical wiring can be produced by peeling the stretchable conductor layer from the underlying substrate and attaching the hot-melt adhesive layer directly to the garment. In this method, the electrical wiring can be provided on the garment without leaving the underlying substrate.

By using the stretchable conductor layer having the electrical wiring and the hot-melt adhesive layer of the present invention, the electrical wiring can be transferred to the fabric, and a garment provided with the electrical wiring can be easily obtained.

EXAMPLES

Hereinafter, the present invention will be described in more detail and specifically with reference to examples. Operations, evaluation results, and the like in the examples were measured by the following methods.

<Preparation Method of Sample for Evaluation>

A polypropylene film (PYLEN OT; 50 $\mu$m thickness) manufactured by Toyobo Co., Ltd. was coated with a resin composition, a resin solution, or a paste using an applicator having a gap of 300 $\mu$m and a width of 130 mm (the coated surface was 130 mm $\times$ 200 mm). The coated film was fixed on cardboard, dried using a hot air dryer at 120°C for 30 minutes, and then cooled to room temperature. Then, the resin film was peeled from the polypropylene film to prepare a sample for evaluation.

<Glass Transition Temperature>

5 mg of the sample for evaluation was placed in an aluminum sample pan and sealed, and the measurement was conducted using a differential scanning calorimeter (DSC) DSC-7020 manufactured by Seiko Instruments Inc., up to 100°C at a temperature increase rate of 20°C/min. The temperature of an intersection of an extended line of a base line and a tangent showing a maximum inclination in a transition part was defined as a glass transition temperature.

<Equilibrium Moisture Absorption Rate and Saturated Water Content>

Five test pieces (samples for evaluation or pieces of the stretchable conductor layer) were prepared. The mass of each test piece after drying at 120°C for 1 hour was measured and taken as an initial mass.

Next, the mass of each test piece left to stand for 24 hours in an environment of 25°C and 60% RH was measured and taken as an equilibrium moisture absorption mass. An equilibrium moisture absorption rate was determined by the following equation:

$$\text{Equilibrium moisture absorption rate} = 100 \times (\text{equilibrium moisture absorption mass} - \text{initial mass}) / \text{initial mass.}$$

Next, the test pieces after the measurement of the equilibrium moisture absorption rate were immersed in water at 40°C, and taken out one by one after 1 hour, 2 hours, 4 hours, 8 hours, and 24 hours to check the water content of each test piece. The water content when the change in the water content did not increase by 1% by mass or more with respect to the initial mass of the test piece was defined as a saturated water content. (Actually, the test piece was almost saturated after immersion for 8 hours.)

As for the water content, after moisture on the outer surface of the sample taken out was removed, the mass in a water-containing state was measured, and the water content was calculated by the following equation:

$$\text{Saturated water content} = 100 \times (\text{mass in water-containing state} - \text{initial mass}) / \text{initial mass.}$$

<Evaluation of Specific Resistance>

The sheet resistance and the thickness of the stretchable conductor layer in a natural state (elongation ratio: 0%) were measured to calculate a specific resistance. The thickness was measured using Thickness Gauge SMD-565L (manufactured by TECLOCK Co., Ltd.), the sheet resistance was measured by Loresta-GP MCP-T610 (manufactured by Mitsubishi Chemical Analytech Co., Ltd.) for four test pieces, and the average value of measured values of each were used.

The specific resistance was calculated by the following equation:

$$\text{Specific resistance } (\Omega \cdot cm) = Rs \ (\Omega/\square) \times t \ (cm).$$

In the equation, Rs represents a sheet resistance measured under each condition, and t represents a thickness measured under each condition.

<Elastic Modulus, Elongation at Break>

A piece with a sample size of 10 mm × 50 mm was cut out from the sample for evaluation or the stretchable conductor layer, clamped by 20 mm from upper and lower sides each on a sample fixing chuck of a tensile tester (RTA-100 manufactured by ORIENTEC CORPORATION), and measured under the conditions of an inter-chuck distance of 10 mm, a tensile speed of 20 mm/min, and a temperature of 25°C and 30 RH%. The elongation at break was determined from the stress-strain curve (S-S curve), and the initial elastic modulus was determined from the initial slope of the stress-strain curve.

The measurement was conducted at five points each for the test piece left to stand for 24 hours in an environment of 25°C and 60% RH (in a state of equilibrium moisture absorption) and the test piece immediately after being immersed in water of 40°C for 24 hours and then taken out (a water-saturated state), and the average value of measured values in each state was found.

<Washing Durability>

According to JIS2017, method 103, 5-times-acceleration test (after washing 5 times consecutively, drying once in the shade), 100 washings were performed in total with a washing net using a household washing machine (ASW-50F5 (HS) manufactured by SANYO Electric Co., Ltd.) and a washing test automatic control machine (SAD-135 type, Tsujii Senki Kogyo Co., Ltd.). The change in resistance for each washing was measured, a case where the resistance reached a value 1000 times or more the initial resistance value was judged to be a "break in wiring" and evaluated as "poor", and a case where the resistance was less than 1000 times the initial resistance value was evaluated as "good". As a detergent, a powder type of Attack was used.

PRODUCTION EXAMPLE

<Resin Production Example 1>

Synthesis of Polyurethane Resin (A)

In a four-necked flask of 1 L, 50 parts of ODX-688 (polyester diol manufactured by DIC), 33 parts of RV220 (manufactured by Toyobo, copolymerized polyester), and 5 parts of neopentyl glycol and 2 parts of 1,6-hexanediol (manufactured by Ube Industries) as chain extenders were placed in 236 parts of diethylene glycol monoethyl ether acetate and 79 parts of ethylene glycol monobutyl ether acetate, and the flask was set in a mantle heater. A stir bar with a stirring seal, a reflux condenser, a temperature detector, and a ball plug were set in the flask, and the materials were dissolved by stirring at 50°C for 30 minutes. 26 parts of diphenylmethane diisocyanate (MDI) and 0.4 parts of dibutyltin dilaurate as a catalyst were added thereto. When the temperature rise due to the reaction heat settled, the temperature was raised to 90°C, and the mixture was allowed to react for 4 hours to obtain a polyurethane resin (A) solution. The aromatic concentration of the polyurethane resin (A) was 45% by mass. The properties of the obtained resin are shown in Table 1.

The aromatic concentration was determined by the following equation:

$$\text{Aromatic concentration (\% by mass)} = \text{total molecular weight of benzene}$$
$$\text{ring part / molecular weight of polyurethane resin.}$$

Here, the molecular weight of a benzene ring part was calculated by assuming that the benzene ring has 12 carbon atoms and 4 hydrogen atoms with respect to one benzene ring.

<Resin Production Example 2>

Synthesis of Polyurethane Resin (B)

In a four-necked flask of 1 L, 100 parts of ODX-2044 (polyester diol manufactured by DIC), 33 parts of P-2010 (polyester diol manufactured by Kuraray), and 30% by weight of 1,6-hexanediol (manufactured by Ube Industries) as a chain extender were placed in 125 parts of diethylene glycol monoethyl ether acetate, and the flask was set in a mantle heater. A stir bar with a stirring seal, a reflux condenser, a temperature detector, and a ball plug were set in the flask, and the materials were dissolved by stirring at 50°C for 30 minutes. 70 parts of Desmodur I (manufactured by Bayer, isocyanate) and 1 part of bismuth metal catalyst were added thereto. When the temperature rise due to the reaction heat settled, the temperature was raised to 90°C, and the mixture was allowed to react for 4 hours to obtain a polyurethane resin (B) solution. The aromatic concentration of the polyurethane resin (B) was 12.7% by mass. The properties of the obtained resin are shown in Table 1.

<Resin Production Example 3>

Synthesis of Polyurethane Resin (C)

In a four-necked flask of 1 L, 400 parts by mass of Coatron KYU-1 (polyester urethane manufactured by Sanyo Chemical, Ltd.) was placed in 240 parts by mass of butyl carbitol, and the flask was set in a mantle heater. A stir bar with a stirring seal, a reflux condenser, a temperature detector, and a ball plug were set in the flask, and the materials were dissolved by stirring at 80 to 100°C for 30 minutes. After confirmation of dissolution, a vacuum pump was attached. The pressure was reduced using the vacuum pump, and solvent displacement was performed by reaction at 80 to 100°C for 4 hours to obtain a polyurethane resin (C) solution dissolved in butyl carbitol. The aromatic concentration of the polyurethane resin (C) was 0% by mass. The properties of the obtained resin are shown in Table 1.

<Flexible Resin 4>

As a flexible resin 4, NBR resin "NIPOL (registered trademark) DN003" manufactured by Zeon Corporation was used. The aromatic concentration of the NBR resin is 0% by mass. For convenience, the NBR resin is placed in the same column as the polyurethane resins and indicated as a resin composition D. The properties of the resin are shown in Table 1.

<Resin Production Examples 5 to 7>

The polyurethane resin (A) and the polyurethane resin (B) were mixed at the ratios shown in Table 1 to obtain flexible resins 5 to 7. The aromatic concentrations and physical properties of the obtained flexible resins are shown in Table 1.

<Resin Production Examples 8 to 10>

The polyurethane resin (A) and the polyurethane resin (C) were mixed at the ratios shown in Table 1 to obtain flexible resins 8 to 10. The aromatic concentrations and physical properties of the obtained flexible resins are shown in Table 1.

[Table 1]

| Polyurethane resin composition | Content of resin (% by mass relative to resin component) | | | | Aromatic concentration | Glass transition temperature | Elastic modulus | Elongation | Water content before immersion (Equilibrium moisture absorption rate) | Water content after immersion at 40°C for 24 hours (Saturated water content) |
|---|---|---|---|---|---|---|---|---|---|---|
| | A | B | C | D (NBR) | (% by mass) | (°C) | (Mpa) | (%) | (% by mass) | (% by mass) |
| Flexible resin 1 | 100 | - | - | - | 45.0 | -3 | 7.7 | 1250 | 1.8 | 1.9 |
| Flexible resin 2 | - | 100 | - | - | 12.7 | -20 | 5.5 | 1180 | 0.9 | 5.8 |
| Flexible resin 3 | - | - | 100 | - | 0.0 | -50 | 1.5 | 1250 | 2.1 | 10.8 |
| Flexible resin 4 | - | - | - | 100 | 0.0 | -10 | 0.7 | 2000 | 1.7 | 20.7 |
| Flexible resin 5 | 5 | 95 | - | - | 14.3 | -19 | 5.7 | 1180 | 1.0 | 4.7 |
| Flexible resin 6 | 10 | 90 | - | - | 15.9 | -18 | 5.8 | 1200 | 1.1 | 4.3 |
| Flexible resin 7 | 30 | 70 | - | - | 21.9 | -15 | 6.1 | 1220 | 1.3 | 3.8 |
| Flexible resin 8 | 20 | - | 80 | - | 9.0 | -42 | 2.3 | 1240 | 2.0 | 7.7 |
| Flexible resin 9 | 25 | - | 75 | - | 11.3 | -42 | 2.6 | 1250 | 1.9 | 6.0 |
| Flexible resin 10 | 30 | - | 70 | - | 13.5 | ·38 | 3.2 | 1230 | 2.0 | 4.8 |

<Production Example of Conductive Paste for Forming Stretchable Conductor>

First, the flexible resin 1 was dissolved in a solvent of half the predetermined amount, and conductive particles 1, a rheology control agent 1, and barium sulfate were added to the resultant solution. After being premixed, the solution was dispersed by a three-roll mill to be turned into a paste, thereby to obtain a conductive paste for forming a stretchable conductor shown in Table 2.

Then, conductive pastes for forming a stretchable conductor shown in Table 2 were obtained in the same manner as above by changing materials.

[Table 2]

| Production Example/ Comparative Production Example | | Production Example 1 | Production Example 2 | Production Example 3 | Production Example 3 | Production Example 5 | Comparative Production Example 1 |
|---|---|---|---|---|---|---|---|
| Paste | | P1 | P2 | P3 | P4 | P5 | P6 |
| Paste composition | Conductive particles | Conductive particles 1 | Conductive particles 1 | Conductive particles 1 | Conductive particles 1 | Conductive particles 2 | Conductive particles 1 |
| | Flexible resin | Flexible resin 1 | Flexible resin 1 | Flexible resin 1 | Flexible resin 1 | Flexible resin 1 | Flexible resin 1 |
| | Rasin/solid content ratio (% by mass) | 16.2 | 15.7 | 17.8 | 20.7 | 15.7 | 11.1 |
| | Solvent 1 | ECA | ECA | ECA | ECA | ECA | ECA |
| | Solvent 2 | BCA | BCA | BCA | BCA | BCA | BCA |
| | Barium sulfate | Added | Not added | Added | Added | Not added | Not added |
| | Rheology control agent | Added | Added | Added | Added | Added | Added |

| Production Example/ Comparative Production Example | | Comparative Production Example 2 | Comparative Production Example 3 | Comparative Production Example 4 | Comparative Production Example 5 | Comparative Production Example 6 | Comparative Production Example 7 |
|---|---|---|---|---|---|---|---|
| Paste | | P7 | P8 | P9 | P10 | P11 | P12 |
| Paste composition | Conductive particles | Conductive particles 1 | Conductive particles 1 | Conductive particles 1 | Conductive particles 1 | Conductive particles 3 | Conductive particles 4 |
| | Flexible resin | Flexible resin 1 | Flexible resin 2 | Flexible resin 3 | Flexible resin 1 | Flexible resin 1 | Flexible resin 4 |
| | Resin/solid content ratio (% by mass) | 12.1 | 15.7 | 15.7 | 37.7 | 32.7 | 14.8 |
| | Solvent 1 | ECA | ECA | BC | ECA | ECA | IS |
| | Solvent 2 | BCA | - | - | BCA | BCA | - |
| | Barium sulfate | Not added | Not added | Not added | Not added | Not added | Added |
| | Rheology control agent | Added | Added | Added | Added | Not added | Not added |

(continued)

| Production Example/ Comparative Production Example | | Production Example 6 | Production Example 7 | Production Example 8 | Comparative Production Example 8 | Comparative Production Example 9 | Production Example 9 |
|---|---|---|---|---|---|---|---|
| Paste | | P13 | P14 | P15 | P17 | P17 | P18 |
| Paste composition | Conductive particles | Conductive particles 1 | Conductive particles 1 | Conductive particles 1 | Conductive particles 1 | Conductive particles 2 | Conductive particles 1 |
| | Flexible resin | Flexible resin 1 | Flexible resin 1 | Flexible resin 1 | Flexible resin 1 | Flexible resin 1 | Flexible resin 1 |
| | Resin/solid content ratio (% by mass) | 16.2 | 16.2 | 16.2 | 16.2 | 16.2 | 16.2 |
| | Solvent 1 | ECA | ECA | ECA | ECA | ECA | ECA |
| | Solvent 2 | BCA | BCA | BCA | BCA | BCA | BCA |
| | Barium sulfate | Added | Added | Added | Added | Added | Added |
| | Rheology control agent | Added | Added | Added | Added | Added | Added |

<Production Example of Paste (Insulating Coating Agent) for Forming Protective

Layer for Forming Insulating Protective Layer>

First, the flexible resin 1 was dissolved in a solvent of half the predetermined amount, and a filler 1 was added to the resultant solution. After being premixed, the solution was dispersed by a three-roll mill to be turned into a paste, thereby to obtain a paste (IP1) for forming a protective layer for forming an insulating protective layer shown in Table 3. Likewise, pastes (IP2) and (IP3) for forming a protective layer for forming an insulating protective layer shown in Table 3 were obtained in the same manner as above by changing the flexible resin.

<Production Example of Paste (Carbon Paste) for Forming Protective Layer for Forming Conductive Protective Layer>

First, the flexible resin 1 and the flexible resin 3 were dissolved in a solvent of half the predetermined amount, and conductive particles 5 were added to the resultant solution. After being premixed, the solution was dispersed by a three-roll mill to be turned into a paste, thereby to obtain conductive pastes (CP1) and (CP2) for forming a protective layer shown in Table 3.

[Table 3]

| Paste for forming a protective layer | | Insulating protective layer (stretchable insulating layer) | | | Conductive protective layer | |
|---|---|---|---|---|---|---|
| | | IP1 | IP2 ' | IP3 | CP1 | CP2 |
| Paste composition | Filler/Conductive particles | Filler 1 | Filler 1 | Filler 1 | Conductive particles 5 | Conductive particles 5 |
| | | | | | | Conductive particles 6 |
| | Filler blending ratio | 100 | 100 | 100 | 100 | 50/50 |
| | Flexible resin | Flexible resin 1 | Flexible resin 2 | Flexible resin 3 | Flexible resin 7 | Flexible resin 10 |
| | Resin/solid content ratio (% by mass) | 45.8 | 45.8 | 45.8 | 64.5 | 77.7 |
| | Solvent 1 | ECA | ECA | ECA | ECA | ECA |
| | Solvent 2 | BCA | BCA | BCA | BC | BCA |
| | Solvent 3 | | | | BCA | - |
| Elastic modulus at equilibrium moisture absorption [MPa] | | 71 | 48 | 23 | - | - |
| Elastic modulus when saturated with water [MPa] | | 65 | 16.2 | 4.7 | - | - |
| (x) Coating film elongation at break at equilibrium moisture absorption [%] | | 350 | 480 | 640 | - | - |
| (y) Coating film elongation at break at the time of saturated water supply [%] | | 400 | 650 | 530 | - | - |
| (y)/(x) ratio | | 1.14 | 1.35 | 0.83 | - | - |

The raw materials in Tables 2 and 3 are as follows. Conductive particles 1: Fine flakey silver powder AGCA, general-purpose type (average particle size: 3 $\mu$m) manufactured by Fukuda Metal Foil & Powder Co., Ltd.

Conductive particles 2: AB5893 (average particle size: 5.0 μm), Metalor Technologies Japan Corporation

Conductive particles 3: Silver-coating powder, general-purpose type (average particle size 1.2 μm) manufactured by Mitsubishi Materials Corporation

Conductive particles 4: Aggregated silver powder G-35 (average particle size: 6.0 μm), DOWA Electronics Materials Co., Ltd.

Conductive particles 5: Graphite powder, Ketchen Black ECP600JD manufactured by Lion Specialty Chemicals Co.,Ltd.

Conductive particles 6: Vein graphite, Graphite BF (average particle size: 5.0 μm) manufactured by Chuetsu Graphite Works Co., Ltd.

Filler 1: B-34 (average particle size 0.3 μm), Sakai Chemical Industry Co., Ltd.

Flexible resin 1: Polyurethane resin A obtained in Resin Production Example 1

Flexible resin 2: Polyurethane resin B obtained in Resin Production Example 2

Flexible resin 3: Polyurethane resin C obtained in Resin Production Example 3

Flexible resin 4: DN003, ultrahigh-nitrile-type, JSR Corporation

Flexible resin 7: Polyurethane resin obtained in Resin Production Example 7

Flexible resin 10: Polyurethane resin obtained in Resin Production Example 10

Rheology control agent 1; MK Conk, Kyoeisha Chemical Co.,Ltd.

Solvent 1: Diethylene glycol monoethyl ether acetate (ECA)

Solvent 2: Ethylene glycol monoethyl ether acetate (BCA)

Solvent 3: Diethylene glycol monobutyl ether (BC)

Solvent 4: Isophorone (IS)

<Production Example and Evaluation of Stretchable Conductor Layer>

A conductive paste for forming a stretchable conductor was applied to a stretchable substrate made of a flexible resin with a thickness of 100 μm using an applicator or a screen printing machine and dried at 100°C for 30 minutes to prepare a stretchable conductor layer (stretchable conductor sheet).

The following physical properties of the obtained stretchable conductor layer (stretchable conductor sheet) are shown in Table 4:

washing durability;

(a) coating film elongation (%) after immersion at 40°C for 24 hours;
(b) coating film elongation (%) in the state of equilibrium moisture absorption; and
(a)/(b) ratio.

[Table 4]

| Example/Comparative Example | | Example 1 | Example 2 | Example 3 | Example 3 | Example 5 | Com. Ex. 1 |
|---|---|---|---|---|---|---|---|
| Paste | | P1 | P2 | P3 | P4 | P5 | P6 |
| Physical properties | Washing durability | Good | Good | Good | Good | Good | Poor |
| | Thickness of stretchable conductor layer (μm) | 40 | 40 | 30 | 30 | 40 | 40 |
| | (a) Coating film elongation (%) after immersion at 40°C for 24 hours | 600 | 600 | 700 | 1000 | 800 | 100 |
| | (b) Coating film elongation (%) in a state of equilibrium moisture absorption | 500 | 500 | 600 | 750 | 600 | 500 |
| | (a)/(b) ratio | 1.20 | 1.20 | 1.17 | 1.33 | 1.33 | 0.20 |

(continued)

| Example/Comparative Example | | Com. Ex. 2 | Com. Ex. 3 | Com. Ex. 4 | Com. Ex. 5 | Com. Ex. 6 | Com. Ex. 7 |
|---|---|---|---|---|---|---|---|
| Paste composition | | P7 | P8 | P9 | P10 | P11 | P12 |
| Physical properties | Washing durability | Poor | Poor | Poor | Poor | Poor | Poor |
| | Thickness of stretchable conductor layer ($\mu$m) | 40 | 40 | 40 | 50 | 50 | 40 |
| | (a) Coating film elongation (%) after immersion at 40°C for 24 hours | 20 | 300 | 250 | 350 | 200 | 300 |
| | (b) Coating film elongation (%) in a state of equilibrium moisture absorption | 200 | 500 | 300 | 200 | 150 | 600 |
| | (a)/(b) ratio | 0.10 | 0.60 | 0.83 | 1.75 | 1.33 | 0.50 |
| Example/Comparative Example | | Example 6 | Example 7 | Example 8 | Com. Ex. 8 | Com. Ex. 9 | Example 9 |
| Paste | | P13 | P14 | P15 | P16 | P17 | P18 |
| Physical properties | Washing durability | Good | Good | Good | Poor | Poor | Good |
| | Thickness of stretchable conductor layer ($\mu$m) | 40 | 40 | 40 | 40 | 40 | 40 |
| | (a) Coating film elongation (%) after immersion at 40°C for 24 hours | 300 | 350 | 400 | 200 | 300 | 300 |
| | (b) Coating film elongation (%) in a state of equilibrium moisture absorption | 450 | 500 | 500 | 350 | 400 | 400 |
| | (a)/(b) ratio | 0.67 | 0.70 | 0.80 | 0.57 | 0.75 | 0.75 |

<Application Example 1: Direct Printing Method>

The paste IP1 for forming a protective layer obtained in the above Production Example of Paste (Insulating Coating Agent) for Forming Protective Layer for Forming Insulating Protective Layer was applied to a release film of a PET substrate and dried until just before the surface tack disappeared. Next, a fabric as a substrate was stacked on the layer of the paste for forming a protective layer and lightly pressure-bonded using a roll laminator. Then. drying was continued, and the release film was peeled off to obtain a fabric having one surface on which an IP1 coating film was formed. This IP1 coating film serves as an insulating base layer (first insulating layer). Almost half of the first insulating layer penetrated into the fabric (substrate), and the sum of thicknesses of the layer that had penetrated and the layer that had not penetrated was 80 $\mu$m. As the fabric, a 2-way tricot cloth KNZ2740 (nylon yarn:urethane yarn = 63%:37% (blend ratio), areal weight: 194 g/m$^2$) manufactured by GUNSEN CO., LTD. was used.

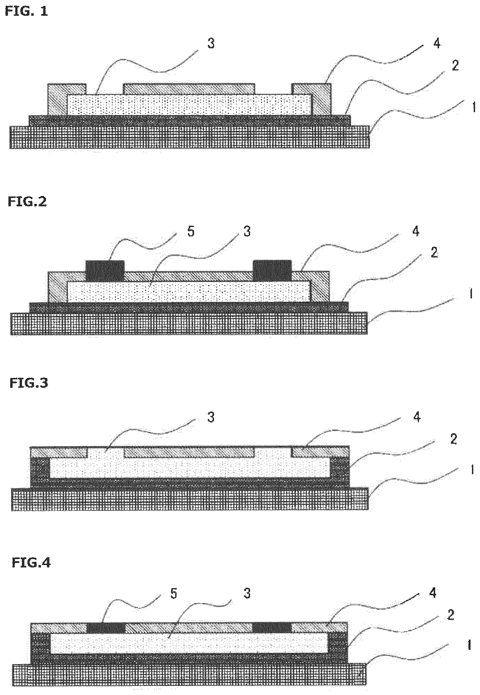

Next, the conductive paste P1 was applied to the first insulating layer with a screen printing machine such that the cross-sectional configuration was as shown in FIG. 1 and dried at 100°C for 30 minutes to form a stretchable conductor layer having a thickness of about 25 $\mu$m. Further, the paste IP1 for forming a protective layer was screen-printed to form a second insulating layer (stretchable cover layer, insulating cover layer), whereby a stretchable conductive trace CT1 having an electrode portion and a wiring portion on the fabric was obtained. Here, in the stretchable conductor layer, the portion that is not covered by the second insulating layer is referred to as an electrode portion for convenience, and the portion that is covered by the second insulating layer is referred to as a wiring portion for convenience.

The washing durability of the obtained stretchable trace was evaluated. The results are shown in Table 5.

<Application Example 2: Direct Printing Method>

On the electrode portion of the conductive trace obtained in Application Example 1, the paste CP1 for forming a conductive protective layer was printed as a conductive protective layer (electrode surface layer, stretchable carbon layer) and dried to obtain a stretchable conductive trace CT2 with a conductive protective layer. The washing durability of the obtained stretchable conductive trace was evaluated. The results are shown in Table 5.

<Application Example 3: Printing Transfer Method>

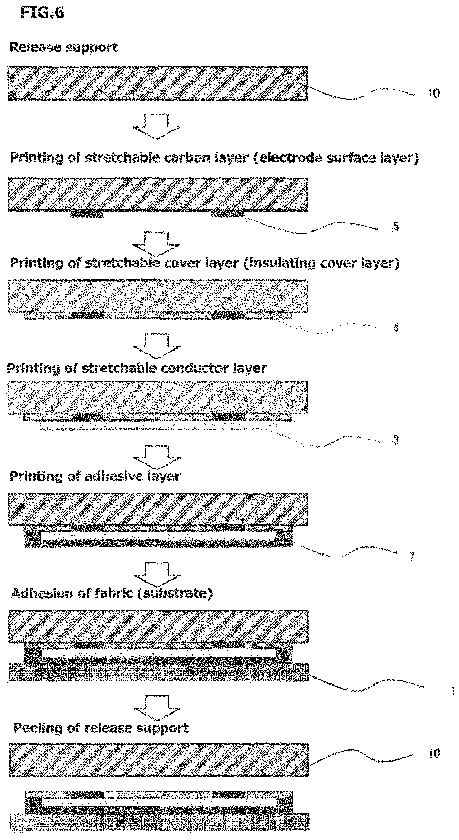

According to the process view of FIG. 6, by operating so as to have the configuration shown in FIG. 4, a stretchable conductive trace CT3 was obtained on a fabric as a substrate by a transfer method. The CP1 was used as a stretchable carbon layer, the IP1 was used as an insulating cover layer, the P1 was used as a stretchable conductor layer, and the IP1 was used as an adhesive layer. The washing durability of the obtained stretchable conductive trace was evaluated. The results are shown in Table 5.

<Application Examples 4 to 6, Application Comparative Examples 1 to 3>

Likewise, conductive traces CT4 to CT9 were obtained using the pastes shown in Table 5. The results are shown in Table 5.

[Table 5]

| | | Application Example 1 | Application Example 2 | Application Example 3 | Application Example 4 | Application Example 5 | Application Example 6 | Application Com. Ex. 1 | Application Com. Ex. 2 | Application Com. Ex. 3 |
|---|---|---|---|---|---|---|---|---|---|---|
| Conductive trace | | CT1 | CT2 | CT3 | CT4 | CT5 | CT6 | CT7 | CT8 | CT9 |
| Process | | Direct printing | Direct printing | Printing transfer | Printing transfer | Printing transfer | Printing transfer | Direct printing | Direct printing | Direct printing |
| Substrate | | GUNSEN 2-way tricot | | | | | | | | |
| First insulating layer (Insulating base layer) (Adhesive layer) | Paste | IP1 | IP1 | IP1 | IP1 | IP1 | IP1 | TP2 | IP3 | IP1 |
| | Thickness ($\mu$m) | 80 | 80 | 30 | 30 | 30 | 30 | 80 | 80 | 80 |
| Stretchable conductor layer | Paste | P1 | P1 | P1 | P13 | P14 | P15 | P13 | P13 | P6 |
| | Thickness ($\mu$m) | 25 | 30 | 25 | 25 | 25 | 25 | 25 | 25 | 25 |
| Second insulating layer (Stretchable cover layer) | Paste | IP1 | IP1 | IP1 | IP1 | IP1 | IP1 | IP2 | IP3 | IP1 |
| | Thickness ($\mu$m) | 25 | 25 | 25 | 25 | 25 | 25 | 25 | 25 | 25 |
| Electrode surface layer | Paste | - | CP1 | CP1 | CP2 | CP2 | CP1 | CP1 | CP1 | CP1 |
| | Thickness ($\mu$m) | - | 15 | 15 | 15 | 15 | 15 | 15 | 15 | 15 |
| Washing durability | | Good | Good | Good | Good | Good | Good | Poor | Poor | Poor |

<Application Examples 7 to 12, Application Comparative Examples 4 to 6>

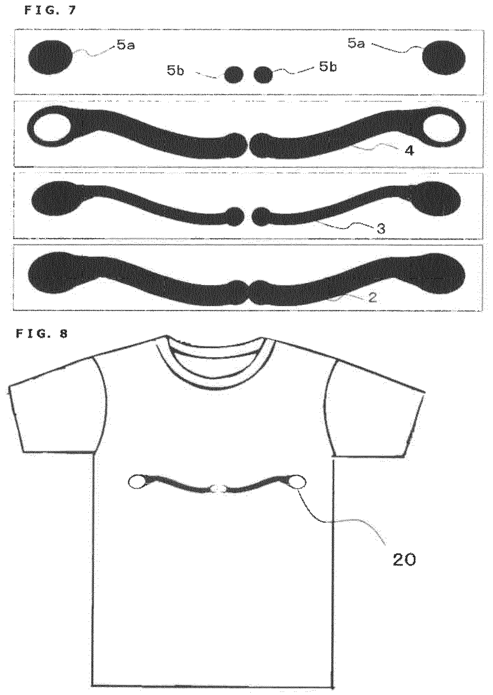

Using a T-shirt made of a 50/50 cotton/polyester blend fabric as a substrate fabric, by the process and materials used in each of Application Examples, an insulating base layer, a stretchable conductor layer, a stretchable cover layer, and an electrode protective layer shown in FIG. 7 were sequentially laminated according to the process to produce a T-shirt in which a conductive trace for measuring an electrocardiogram was arranged. The combination of materials and the process are shown in Table 6.

The T-shirt has a girth adjustment mechanism by means of a hook-and-loop fastener in an underarm portion. By adjusting the position of the hook-and-loop fastener after putting on the T-shirt, an electrode for measuring an electrocardiogram can be adjusted to come into contact with a skin. To a connector connection section 5b, a snap fastener as a connector was attached, and a heart rate sensor WHS-2 manufactured by Union Tool Co., which has a wireless data transmission function to a smartphone, was connected as a detachable electronic unit. The heart rate sensor WHS-2 was set so that an electrocardiogram signal was sent to a smartphone from WHS-2 simultaneously with measurement, and the heart rate data was received with a smartphone manufactured by Apple Inc. incorporating the app "my Beat" dedicated to the heart rate sensor WHS-2 and displayed on the screen, and further RRI (ms (millisecond)) and HR (heart rate (beats/minute)) were able to be measured. Incidentally, RRI is the time interval between the R wave, which is the wave with the largest potential difference in the electrocardiogram, and the next R wave, and (heart rate) = 60 / (R - R time (seconds)) [bpm] can be calculated from RRI. As described above, an electrocardiogram information measurement system using a biological information measurement garment having a heart rate measurement function was configured.

<Wearing and Exercise Test>

A subject was allowed to wear each biological information measurement garment obtained in Application Examples 7 to 12 and Application Comparative Examples 4 to 6 and perform continuously radio calisthenics No. 1 and radio calisthenics No. 2, and RRI and HR were measured during that time. The test conditions are as follows.

- Test environment: 20°C 50% (At the end of the operation protocol, the subject is not sweating).

- Press the electrode portion by hand at the start of the protocol, and then do not hold it by hand until the end.

- Measure the garment pressure on the electrode portion and the armpit before starting and make adjustment so that the pressure of the electrode portion is 0.8 $\pm$ 0.05 kPa, and the pressure of the armpit is 0.7 $\pm$ 0.05 kPa.

As a result, RRI and HR were able to be obtained satisfactorily in any biological information measurement garment.

<Washing Durability of Biological Information Measurement Garment>

After the wearing and exercise test, the washing durability test of the biological information measurement garment was conducted. The results are shown in Table 6.

[Table 6]

| | | Application Example 7 | Application Example 8 | Application Example 9 | Application Example 10 | Application Example 11 | Application Example 12 | Application Com. Ex. 4 | Application Com. Ex. 5 | l Application Com. Ex. 6 |
|---|---|---|---|---|---|---|---|---|---|---|
| Conductive trace | | CT10 | CT11 | CT12 | CT13 | CT14 | CT15 | CT16 | CT17 | CT18 |
| Process | | Direct printing | Direct printing | Printing transfer | Printing transfer | Printing transfer | Printing transfer | Direct printing | Direct printing | Direct printing |
| | | | | | | | | | | |
| Substrate | | 50/50 cotton/polyester blend fabric | | | | | | | | |
| First insulating layer (Insulating base layer) (Adhesive layer) | Paste | IP1 | IP1 | IP1 | IP1 | IP1 | IP1 | IP2 | IP3 | IP1 |
| | Thickness ($\mu$m) | 100 | 120 | 30 | 30 | 30 | 30 | 120 | 120 | 120 |
| Stretchable conductor layer | Paste | P1 | P1 | P1 | P13 | P14 | P15 | P13 | P13 | P6 |
| | Thickness ($\mu$m) | 35 | 35 | 25 | 25 | 25 | 25 | 35 | 35 | 35 |
| Second insulating layer (Stretchable cover layer) | Paste | IP1 | IP1 | IP1 | IP1 | IP1 | IP1 | IP2 | IP3 | IP1 |
| | Thickness ($\mu$m) | 30 | 30 | 25 | 25 | 25 | 25 | 30 | 30 | 30 |
| Electrode surface layer | Paste | - | CP1 | CP1 | CP2 | CP2 | CP1 | CP1 | CP1 | CP1 |
| | Thickness ($\mu$m) | - | 20 | 15 | 15 | 15 | 15 | 20 | 20 | 20 |
| Washing durability | | Good | Good | Good | Good | Good | Good | Poor | Poor | Poor |

<Trial Example Using Stretchable Polymer Film as Substrate>

Using a polyurethane sheet DUS605-CD PT5S manufactured by Sheedom Co.,Ltd. as a substrate, the conductive paste P1 was applied thereto with a screen printing machine such that the cross-sectional configuration was as shown in FIG. 2 and dried at 100°C for 30 minutes to form a stretchable conductor layer having a thickness of about 25 μm. Further, the paste IP1 for forming a protective layer was screen-printed thereon to form a second insulating layer (stretchable cover layer, insulating cover layer), and further, the paste CP2 for forming a conductive protective layer was used to form an electrode protective layer, whereby a conductive trace CT19 was obtained. Likewise, using a polyurethane sheet TG88K manufactured by Formance Co., Ltd., the operations were performed to obtain a conductive trace CT20. The evaluation results on the washing durability of both CT19 and CT20 were "good".

< Trial Example Using Polyurethane Sheet with Hot-Melt>

By the same operations and the same configuration as in CT19, an elastomer sheet with a conductive trace CT21 having the shape shown in FIG. 7 was obtained on a non-hot-melt layer side of "MOBILON" manufactured by Nisshinbo Holdings Inc., which is an elastomer sheet with a hot-melt layer. Then, a hot-melt layer side of the elastomer sheet was placed face-to face with the reverse side (the side that comes into contact with a skin when being worn) of a compression-type T-shirt, the elastomer sheet and the compression-type T-shirt were sandwiched between fluororesin sheets and hot-pressed at 140 ± 30 seconds, thereby to obtain a compression type T-shirt with the conductive trace CT21, which is a biological information measurement garment having an electrocardiogram measurement function. A snap fastener was attached to the obtained T-shirt in the same manner as in Application Example 7, then likewise, a heart rate sensor WHS-2 manufactured by Union Tool Co. was connected, and the wearing and exercise test was conducted. The T-shirt had no problem with the electrocardiogram measurement function, and RRI and HR was able to be measured satisfactorily. The washing durability test was conducted after wearing of the T-shirt, and the test result was "good".
Likewise, using a polyurethane hot-melt film Ecellent SHM104-PUR (with a separator) manufactured by NTW Co., Ltd. as a substrate, a conductive trace 22 was obtained with the same material composition and process as above, and the obtained conductive trace was similarly attached to a compression type T-shirt by means of a hot press, thereby to obtain a biological information measurement garment having an electrocardiogram measurement function. Likewise, the wearing and exercise test and the washing durability test were conducted, and the results of the tests were good.

INDUSTRIAL APPLICABILITY

As described above, the conductive paste for forming a stretchable conductor, the stretchable conductor layer, the method for producing a stretchable conductor layer, the stretchable electrical wiring structure, and the biological information measurement device of the present invention are capable of forming an electrode or electrical wiring having excellent washing durability, and are extremely useful as an electrical wiring material and electrical insulating material in the production of a garment-type biological information measurement device.
The stretchable conductive coating film of the present invention is, by using it in a wearable smart device, applicable to a wearable device for detecting information of a human body such as bioelectric potential including myoelectric potential and cardiac potential, and biological information including body temperature, pulse, blood pressure, and the like by a sensor or the like provided in a garment; a garment incorporating an electric heating device; a wearable device incorporating a sensor for measuring a clothing pressure; wear that measures a body size by using a clothing pressure; a sock-type device for measuring a pressure on a sole of foot; a garment in which flexible solar cell modules are integrated in textiles; a wiring part of a tent, bag or the like; a wiring part of a low-frequency treatment device with a joint, a thermotherapy machine, or the like; a sensing unit of degree of flexion, and the like. Such a wearable device can be applied not only to a human body but also to an animal such as pet or livestock, and a mechanical device having a stretching portion, a bending portion, and the like, and it can also be used as an electrical wiring of a system that is used by connecting a mechanical device such as a robotic prosthetic arm or leg to a human body. In addition, it is also useful as a wiring material for an implant device to be embedded in the body. Furthermore, it can be widely used as a conductive trace for automotive use, industrial machinery, home appliances, and the like, without being limited to living bodies.

REFERENCE SIGNS LIST

1:    Substrate (fabric)
2:    Insulating base layer

3:      Stretchable conductor layer (stretchable conductor layer)
4:      Stretchable cover layer (insulating cover layer, second insulating layer)
5:      Stretchable carbon layer (electrode surface layer)
5a:     Skin contact electrode section
5b:     Connector connection section
6:      Adhesive layer (insulating base layer, first insulating layer)
10:     Temporary support (release support)
20:     Stretchable electrode portion and stretchable wiring portion for measuring an electrocardiogram

**Claims**

1.  A conductive paste for forming a stretchable conductor, the conductive paste comprising conductive particles and a flexible resin,

    wherein the flexible resin has a saturated water content of 5% by mass or less, and
    wherein the flexible resin is contained in an amount of 13% by mass to 35% by mass relative to total solids content in the conductive paste for forming a stretchable conductor.

2.  The conductive paste for forming a stretchable conductor according to claim 1, wherein the flexible resin is a non-crosslinked polyurethane resin.

3.  The conductive paste for forming a stretchable conductor according to claim 1 or 2, wherein the flexible resin has a glass transition temperature of —10°C or higher and +10°C or lower.

4.  A stretchable conductor layer obtained by printing or applying the conductive paste for forming a stretchable conductor according to any one of claims 1 to 3 on or to a substrate, followed by drying,
    wherein an elongation at break ($\varepsilon$') in a water-saturated state at 40°C of the stretchable conductor layer is 500% or more.

5.  The stretchable conductor layer according to claim 4,
    wherein an elongation at break ($\varepsilon$) in a state of equilibrium moisture absorption of the stretchable conductor layer and the elongation at break ($\varepsilon$') in a water-saturated state at 40°C of the stretchable conductor layer satisfy a relationship of $\varepsilon$' / $\varepsilon$ > 0.5.

6.  A method for producing a stretchable conductor layer, comprising:
    printing or applying the conductive paste for forming a stretchable conductor according to any one of claims 1 to 3 on or to a stretchable substrate, followed by drying at a temperature of at least 70°C or higher and 120°C or lower, to obtain a stretchable conductor layer having a saturated water content of 5% by mass or less.

7.  A method for producing a stretchable conductor layer, comprising:
    printing or applying the conductive paste for forming a stretchable conductor according to any one of claims 1 to 3 on or to a non-stretchable release substrate, followed by drying at a temperature of at least 70°C or higher and 120°C or lower, to obtain a stretchable conductor layer having a saturated water content of 5% by mass or less.

8.  A stretchable electrical wiring structure comprising: a substrate comprising a fiber structure, a stretchable electrode configured to directly contact a living body surface, a stretchable electrical wiring, and a stretchable insulating layer, wherein the stretchable insulating layer comprises a flexible resin composition having a saturated water content of 5% by mass or less and an elastic modulus of 10 MPa or more and 1000 MPa or less when saturated with water.

9.  The stretchable electrical wiring structure according to claim 8, wherein the stretchable insulating layer is formed on a side facing a living body surface of the stretchable electrical wiring structure.

10. The stretchable electrical wiring structure according to claim 8 or 9, wherein the stretchable insulating layer is formed between the stretchable electrode and/or the stretchable electrical wiring and the substrate comprising the fiber structure.

11. The stretchable electrical wiring structure according to any one of claims 8 to 10, wherein the stretchable insulating layer is formed on a surface opposite to a surface, of the substrate comprising the fiber structure, on which the stretchable electrode and/or the stretchable electrical wiring are formed.

12. The stretchable electrical wiring structure according to any one of claims 8 to 11, wherein a stretchable conductor layer constituting the stretchable electrode and/or the stretchable electrical wiring has a saturated water content of 5% by mass or less.

13. The stretchable electrical wiring structure according to any one of claims 8 to 12,

   wherein the stretchable conductor layer comprises conductive particles and a flexible resin,
   wherein the flexible resin has a saturated water content of 5% by mass or less, and
   wherein the flexible resin is contained in an amount of 13% by mass to 35% by mass relative to the stretchable conductor layer.

14. The stretchable electrical wiring structure according to any one of claims 8 to 13, wherein the flexible resin has a glass transition temperature of -10°C or higher and 10°C or lower.

15. The stretchable electrical wiring structure according to any one of claims 8 to 14, wherein an elongation at break ($\varepsilon$') in a water-saturated state at 40°C of the stretchable insulating layer is 500% or more.

16. The stretchable electrical wiring structure according to any one of claims 8 to 15, wherein an elongation at break ($\varepsilon$) in a state of equilibrium moisture absorption of the stretchable insulating layer and the elongation at break ($\varepsilon$') in a water-saturated state at 40°C of the stretchable insulating layer satisfy a relationship of $\varepsilon$' / $\varepsilon$ > 0.5.

17. The stretchable electrical wiring structure according to any one of claims 8 to 16, wherein the flexible resin is a non-crosslinked polyurethane resin.

18. The stretchable electrical wiring structure according to any one of claims 8 to 17, wherein the flexible resin is a polyester-based or polycarbonate-based polyurethane resin containing 13% by mass or more of an aromatic group in a resin skeleton.

19. A biological information measurement device comprising a stretchable electrode configured to directly contact a living body surface,
   wherein a stretchable conductor layer constituting the stretchable electrode is the stretchable conductor layer according to claim 4 or 5.

20. The biological information measurement device according to claim 19, wherein a stretchable electrical wiring is formed by a same stretchable conductor layer as the stretchable conductor layer constituting the stretchable electrode.

21. The biological information measurement device according to claim 19,
   wherein the biological information measurement device comprises a conductive protective film comprising a carbon-based filler as a conductive component on a surface of the stretchable electrode.

22. The biological information measurement device according to any one of claims 19 to 21,
   wherein the biological information measurement device comprises a layer comprising a flexible resin on a surface opposite to a contact surface with a living body surface of the stretchable electrode and/or the stretchable electrical wiring, the flexible resin having a saturated water content of 5% by mass or less and an elastic modulus of 10 MPa or more and 1000 MPa or less in a range of water content of 0 to 5% by mass.

23. The biological information measurement device according to any one of claims 20 to 22,
   wherein the biological information measurement device comprises an insulating layer comprising a flexible resin on a contact surface with a living body surface of the stretchable electrical wiring, the flexible resin having a saturated water content of 5% by mass or less and an elastic modulus of 10 MPa or more and 1000 MPa or less in a range of water content of 0 to 5% by mass.

FIG. 1

FIG.2

FIG.3

FIG.4

# FIG.5

Temporary support (release support)      10

Temporary fixing of fabric (substrate)      1

Printing of stretchable conductor layer      3

Printing of stretchable cover layer (insulating cover layer)      4

Printing of stretchable carbon layer

(electrode surface layer)      5

Peeling of temporary support      10

## FIG.6

Release support

10

Printing of stretchable carbon layer (electrode surface layer)

5

Printing of stretchable cover layer (insulating cover layer)

4

Printing of stretchable conductor layer

3

Printing of adhesive layer

7

Adhesion of fabric (substrate)

1

Peeling of release support

10

FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5363592 B
- JP 5486268 B
- WO 2017154726 A
- WO 2015119217 A